# EUROPÄISCHE PATENTANMELDUNG

(11) **EP 2 127 643 A1**
(43) Veröffentlichungstag der Anmeldung: **02.12.2009**
(21) Anmeldenummer: 08075532.5
(22) Anmeldetag: 30.05.2008
(51) Int. Cl.: A61K 9/50, A61K 31/565

(54) **Orale Pelletformulierungen mit zeitversetzter Wirkstofffreigabe**

(71) Anmelder: Bayer Schering Pharma Aktiengesellschaft, 13353 Berlin (DE)
(72) Erfinder: Lobback, Carmen, 15537 Erkner (DE); Backensfeld, Thomas Dr., 13127 Berlin (DE); Funke, Adrian Dr., 14055 Berlin (DE); Schulze, Julia Dr., 10407 Berlin (DE); Lienau, Philip Dr., 10997 Berlin (DE); Reichel, Andreas Dr., 10179 Berlin (DE)

(57) **Zusammenfassung**

Die Erfindung betrifft eine pharmazeutische Zubereitung, enthaltend einen Wirkstoff und einen funktionellen Hilfsstoff, wobei der Wirkstoff nach oraler Applikation der Zubereitung zeitlich verzögert zum funktionellen Hilfsstoff freigesetzt wird. Ferner betrifft die vorliegende Erfindung die Verwendung der erfindungsgemäßen Zubereitung als perorales Arzneimittel.

## Beschreibung

Die Erfindung betrifft eine pharmazeutische Zubereitung, enthaltend einen Wirkstoff und einen funktionellen Hilfsstoff, wobei der Wirkstoff nach oraler Applikation der Zubereitung zeitlich verzögert zum funktionellen Hilfsstoff freigesetzt wird. Ferner betrifft die vorliegende Erfindung die Verwendung der erfindungsgemäßen Zubereitung als perorales Arzneimittel.

Die Bioverfügbarkeit ist eine wichtige Kenngröße eines Wirkstoffes, die durch das Ausmaß und die Geschwindigkeit definiert wird, mit der ein Wirkstoff aus einer pharmazeutischen Formulierung absorbiert wird und am Wirkort oder im systemischen Kreislauf vorliegt (Committee for proprietary medicinal products (CPMP). Note for Guidance on the Investigation of Bioavailability and Bioequivalence. 2001). Ein Maß für die Bioverfügbarkeit ist die Plasmakonzentration zu verschiedenen Zeitpunkten nach oraler Gabe des Wirkstoffes.
Die meisten Arzneimittel werden oral verabreicht, da dies eine kostengünstige und für den Patienten angenehme Applikationsart und darüber hinaus leicht zu handhaben ist (B. Abrahamsson, H.Lennernäs, Application of the biopharmaceutic classification system now and in the future. In H.Waterbeemd, H.Lennernäs and P.Artursson (eds.), Drug Bioavailability, Estimation of Solubility, Permeability, Absorption and Bioavailability, Wiley-VHC, New York, USA, 2005, PP: 495-531; G.Liu, et al., J Clin Oncol, 15 (1):110-115 (1997)). Die orale Bioverfügbarkeit ist daher ein Schlüsselfaktor für den therapeutischen Erfolg.

Das Ausmaß und die Geschwindigkeit der Absorption von Wirkstoffen aus dem Gastrointestinaltrakt hängt von einer Vielzahl von Faktoren ab (S.Hurst, et al., Expert Opinion on Drug Metabolism & Toxicology, 3 (4):469-489 (2007)). Dabei sind die wichtigsten physiko-chemischen Eigenschaften eines Wirkstoffes die Lipophilie, der lonisationsgrad, die Kristallinität und damit einhergehend die Löslichkeit in wässrigen Medien bei physiologischen pH-Werten. Daneben nehmen auch physiologische Faktoren Einfluss auf die Absorption eines Wirkstoffs.

Zusätzlich zur Absorption können sowohl intestinale metabolisierende Enzyme als auch Efflux-Transportsysteme die orale Bioverfügbarkeit von Wirkstoffen entscheidend beeinflussen. Eine Limitierung der systemischen Verfügbarkeit von Wirkstoffen wird durch die intestinale Metabolisierung von Wirkstoffen beziehungsweise durch lumenwärts gerichteteTransportphänomene verursacht.
So können selbst Wirkstoffe, die vollständig aus dem Gastrointestinaltrakt absorbiert werden, infolge dieser intestinalen Enzyme und Effluxsysteme eine stark erniedrigte orale Bioverfügbarkeit aufweisen.

Zahlreiche der auf dem Markt befindlichen Wirkstoffe, beispielsweise Estrogene, verfügen über eine deutlich reduzierte orale Bioverfügbarkeit (Mashchak CA, Lobo RA, Dozono-Takano R, Eggena P, Nakamura RM, Brenner PF and Mishell DR Jr (1982). Um die orale Bioverfügbarkeit zu verbessern, wurden natürliche Estrogene durch Derivatisierung abgewandelt. Konventionelle chemisch modifizierte Estrogene wie z. B. das Ethinylestradiol werden zwar nach oraler Applikation rasch und vollständig absorbiert ( W.L.Chiou, A.Barve, Pharmaceutical Research, V15 (11):1792-1795 (1998); M.J.Reed, K.Fotherby, Journal of Steroid Biochemistry, 11 (2):1107-1112 (1979)), unterliegen jedoch einem starken First-Pass-Effekt, dessen Ausmaß individuell stark variieren kann.

So beträgt die humane absolute Bioverfügbarkeit von Ethinylestradiol ungefähr 45 % (W.Kuhnz et al. Pharmacokinetics of Exogenous Natural and Synthetic Estrogens and Antiestrogens. In M.Oettel and E.Schillinger (eds.), Handbook of Experimental Pharmacology, Springer-Verlag Berlin Heidelberg, 1999, PP: 261-321; M.L.Orme et al., Pharmacology & Therapeutics, 43 (2):251-260 (1989);K.Fotherby, Journal of Steroid Biochemistry, 19 (1, Part 3):817-820 (1983)). Neben hepatischer Glucuronidierung und Hydroxylierung findet während der ersten Darmpassage von Ethinylestradiol ein beträchtlicher Abbau durch intestinale Enzyme wie CYP 3A4, 2C9 und 2C19 ( T. Ebner et al., Mol Pharmacol, 43 (4):649-654 (1993); G.M.Pacifici, D.J.Back, Journal of Steroid Biochemistry, 31 (3):345-349 (1988); B.Wanget al., Drug Metab Dispos, 32 (11):1209-1212 (2004)) statt. 65 % des beobachteten First-Pass-Effekts ist auf die direkte Konjugation von Ethinylestradiol vor allem durch Sulfatierung in der Darmwand zurückzuführen.

**Aus dem Stand der Technik sind verschiedene Möglichkeiten bekannt, intestinale metabolisierende Enzymsysteme beziehungsweise Efflux-Systeme zu inhibieren, um eine Erhöhung der Bioverfügbarkeit von Wirkstoffen zu erreichen.**

Die orale Bioverfügbarkeit von Wirkstoffen, die Substrate für metabolisierende Enzyme und/ oder Efflux-Transporter sind, kann entweder
- durch simultane Verabreichung eines Inhibitors dieser Enzym- und/ oder Effluxsysteme (S.-F.Zhouet al., Ther Drug Monit, 29 (6):687-710 (2007)) oder
- durch einen separat zum oral zu verabreichenden Wirkstoff applizierten Inhibitor erhöht werden (T.R.Buggins et al., Advanced Drug Delivery Reviews, 59 (15):1482-1503 (2007)).

Der simultan oder separat zu verabreichende Inhibitor der intestinalen metabolisierenden Enzymsysteme und/ oder Effluxsysteme ist entweder ein zusätzlicher Wirkstoff oder ein pharmazeutischer Hilfsstoff. Die Applikation eines weiteren Wirkstoffes allein zum Zwecke der Erhöhung der oralen Bioverfügbarkeit des den therapeutischen Effekt erzielenden Wirkstoffs ist allerdings mit einer zusätzlichen systemischen Belastung des Organismus verbunden und daher keine optimale Lösung.

Aus WO 20041032897 sind pharmazeutische Zubereitungen bekannt, die mindestens einen Emulgator, mindestens einen Hilfsemulgator und/ oder Solvent sowie mindestens ein Lipid enthalten, die dadurch gekennzeichnet sind, dass das Massenverhältnis von Emulgator zu Hilfsemulgator und/ oder Solvent (Smix) 1:1 bis 9:1 und der Gesamtlipidanteil > 0 % (m/m) beträgt, wobei diese Zubereitung mindestens ein intestinales Enzym und/ oder mindestens ein intestinales Effluxsystem zumindest teilweise inhibiert. Diese Zubereitungen können verwendet werden, um die Bioverfügbarkeit von Arzneistoffen, die lipophil und/ oder Substrate von intestinalen metabolisierenden Enzymen und/ oder intestinalen Effluxsystemen sind, insbesondere Steroide, zu erhöhen. Diese Zubereitungen, auch "Enzym-Modulierendes-Selbst-Emulgierendes-System (EMSES)" genannt, heben insbesondere auf den pharmakokinetisch zu beeinflussenden Effekt der in der Formulierung enthaltenden Hilfsstoffe ab. Über eine zeitliche Abfolge von Inhibition der Enzyme und/ oder Transporter und Absorption des Wirkstoffs wird keine Aussage getroffen.

Choi et al. berichten über eine zeitversetzte Gabe des Flavonoids Morin, weiches Inhibitor von Pgp und CYP 3A4 ist, und eines Wirkstoffs. 30 Minuten nach oraler Applikation von Morin in Ratten wird Paclitaxel oral gegeben. Dadurch wird die Pharmakokinetik von Paclitaxel (Pgp-Substrat) derart verändert, dass es zu einer Erhöhung der AUC bis zu 70 % kommt. Nach i.v. Gabe von Morin und Paclitaxel ist dagegen kein Effekt zu beobachten war. Dies lässt auf einen Inhibitionseffekt von Morin im Gastrointestinaltrakt bei der Absorption des Paclitaxels schließen (Int. J. Pharm, 323 (1-2):81-85 (2006).

In dem Fertigarzneimittelprodukt Kaletra^{®} zur Behandlung von HIV wird mit dem Hauptwirkstoff Lopinavir, welches einem hohen enteralen First-Pass-Metabolismus sowie dem Efflux durch P-gp unterliegt, Ritonavir als zweiter Wirkstoff zugesetzt (Rote Liste, 2002). Ritonavir, welches in einer Konzentration von 1/6 seiner therapeutischen Dosis eingesetzt wird, verringert die enterale und wahrscheinlich auch die hepatische Metabolisierung und den P-gp-vermittelten Auswärtstransport des Lopinavirs.

Weiterhin sind aus der Literatur Untersuchungen bekannt, bei denen Wirkstoffe, die ausschließlich Pgp-Substrate sind, zusammen mit einem weiteren Wirkstoff zur Inhibition der Pgp-Effluxtransporter verabreicht werden.
Baluom et al. berichten über Tablettenformulierungen, die einen Pgp-Inhibitor wie Verapamil oder Quinidine synchron zu einem Pgp-Substrat wie Sulpirid freisetzen. Die orale Bioverfügbarkeit von Sulpirid konnte so gesteigert werden (J. Controlled Release, 70 (1-2):139-147 (2001).
Terwogt et al. verwendeten in einer Humanstudie den Wirkstoff Cyclosporin als Pgp-Inhibitor. Während oral verabreichtes Paclitaxel als Pgp-Substrat eine geringe Bioverfügbarkeit aufweist, war die Bioverfügbarkeit des Paclitaxels nach gleichzeitiger Gabe von Pgp-Substrat (Paclitaxel) und Pgp-Inhibitor (Cyclosporin) um Faktor 9 erhöht (Lancet, 352 (9124):285 (1998)).

Es sind auch Versuche bekannt, das Absorptionsverhalten von Pgp-Substraten durch verschiedene Hilfsstoffe zu beeinflussen (Cornaire et al., Int. J. Pharm., 278 (1):119-131 (2004). Obwohl durch Coadministration von Hilfsstoff und Pgp-Substrat, hier die Substrate Celiprolol und Digoxin, in vitro eine Erhöhung der Absorption der Wirkstoffe erreicht wurde, konnte in vivo die AUC (= Fläche unter der Plasmakonzentrations-Zeit-Kurve; Maß für die Menge des Arzneistoffes im Körper vom Zeitpunkt der Applikation bis zum Zeitpunkt unendlich) nicht erhöht werden.

Substrate von intestinalen metabolisierenden Enzyme, wie beispielsweise Nifedipin, wurden von Ho et al. untersucht (J. Controlled Release, 68 (3):433-440 (2000). Es wird der Einfluss des Hilfsstoffs Pluronic F-68 auf das Freisetzungsverhalten und die Bioverfügbarkeit von Multilayerpellets beschrieben, die den Wirkstoff Nifedipin in zwei Schichten enthalten. Dabei wurde eine Verbesserung des Freisetzungsverhaltens von Nifedipin durch Pluronic F-68 in vitro beobachtet, eine Verbesserung der oralen Bioverfügbarkeit in vivo wurde jedoch ebenfalls nicht erreicht.

In EP 1 787 640 A1 werden pharmazeutische Formulierungen in Partikelform mit Multilayerstruktur beschrieben, die eine Verzögerung der Wirkstofffreisetzung nach wenigen Minuten bewirken, den Wirkstoff danach aber rasch freisetzen. Ziel einer solch verzögerten Wirkstofffreisetzung ist beispielsweise eine Geschmacksmaskierung und Verhinderung einer zu frühen Absorption des Wirkstoffs in der Mundhöhle.

Zusammenfassend wird festgestellt, dass eine zeitliche Steuerung der Freigabe von Substrat und Inhibitor von intestinalen metabolisierenden Enzymen und/ oder intestinalen Effluxsystemen bei gleichzeitiger Verbesserung der oralen Bioverfügbarkeit aus dem Stand der Technik nicht bekannt ist.

Aufgabe der vorliegenden Erfindung ist es daher, eine definierte, zeitlich verzögerte Freisetzung von Wirkstoffen gegenüber funktionellen Hilfsstoffen aus einer peroral zu applizierenden pharmazeutischen Formulierung zu ermöglichen. Dabei sollen solche Wirkstoffe in der pharmazeutischen Zubereitung formuliert sein, die Substrat von intestinalen metabolisierenden Enzymen sind, wie beispielsweise Estrogene. Als funktionelle Hilfsstoffe sollen solche für die pharmazeutischen Formulierungen verwendet werden, die intestinale metabolisierende Enzyme inhibieren.

Diese Aufgabe wird durch die erfindungsgemäßen festen pharmazeutischen Zubereitungen in Form von sogenannten Multilayerpellets gelöst, die - von innen nach außen gesehen - enthalten:
a) einen Pelletkern,
b) eine Schicht, enthaltend mindestens einen Wirkstoff (= Substrat),
c) eine darüber gelegene Schicht, enthaltend mindestens ein Polymer und
d) eine äußere Schacht, enthaltend mindestens einen funkitonellen Hifsstoff (= Inhibitor).

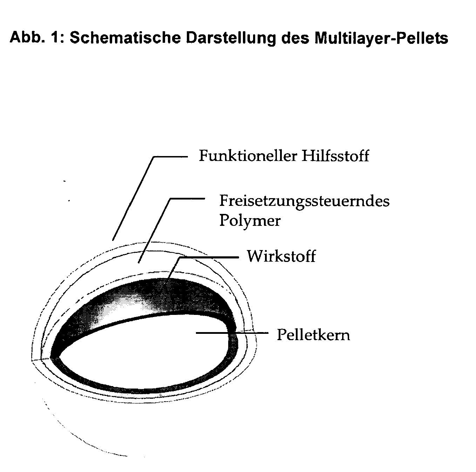

### Siehe Abbildung 1 Schematische Darstellung des Multilayer-Pellets.

Weiterhin umfasst die vorliegende Erfindung die erfindungsgemäßen festen pharmazeutischen Zubereitungen zur Verwendung als peroral zu applizierende Arzneimittel.

Die vorliegende Erfindung bezieht sich auch auf die Verwendung der erfindungsgemäßen festen pharmazeutischen Zubereitung zur Herstellung eines peroral zu applizierenden Arzneimittels zur Behandlung von estrogenabhängigen Erkrankungen wie Ovarialinsuffizienz oder auch zur Verwendung in der weiblichen Fertilitätskontrolle (hormonelle Kontrazeption) oder der weiblichen Hormonersatztherapie.

Ein Vorteil der erfindungsgemäßen Formulierung besteht darin, dass funktionelle Hilfsstoffe sofort freigesetzt werden, um zuerst intestinale metabolisierende Enzyme zu inhibieren. Erst nach Freisetzung der inhibierenden Hilfsstoffe werden die Wirkstoffe aus der Formulierung freigegeben.
Diese Freisetzungsverzögerung von Wirkstoff zum funktionellen Hilfsstoff hat zum Vorteil, dass ein Zeitfenster für die inhibierende Wirkung des funktionellen Hilfsstoffs auf intestinale metabolisierende Enzyme zur Verfügung steht, bevor der Wirkstoff, welcher Substrat für intestinale metabolisierende Enzyme ist, aus der Formulierung freigegeben wird. Dadurch ist gewährleistet, dass der Wirkstoff genau dann auf die intestinalen metabolisierenden Enzyme trifft, wenn diese gerade durch den funktionellen Hilfsstoff inhibiert sind.

Ein weiterer Vorteil der vorliegenden erfindungsgemäßen Formulierung ist ferner, dass für die Inhibition der intestinalen metabolisierenden Enzyme kein weiterer zusätzlicher Wirkstoff benötigt wird, der unerwünschte Nebenwirkungen auslösen kann.
Die Kombination eines Wirkstoffes mit einem zweiten Wirkstoff zum Zweck der Verbesserung der oralen Bioverfügbarkeit wird von den Zulassungsbehörden nur für die Behandlung schwerwiegender Indikationen zugelassen wie etwa für die Behandlung von HIV (siehe weiter oben Kaletra) oder von onkologischen Erkrankungen. Für die Zulassung von Kontrazeptiva beispielsweise wird eine derartige Kombination nicht akzeptiert.
Die inhibierende Wirkung wird erfindungsgemäß mit einem pharmazeutischen Hilfsstoffe erzielt.

Weiterhin vorteilhaft ist, dass eine Kombination von funktionellem Hilfsstoff und Wirkstoff in einer Formulierung realisiert wird. Eine separate, zeitlich aufeinanderfolgende Einnahme von funktionellen Hilfsstoffen und Wirkstoffen ist daher nicht notwendig. Dies führt zusätzlich zu einer höheren Akzeptanz derartig formulierter pharmazeutischer Zubereitungen bei den Patienten.

Ein weiterer Vorteil der Erfindung besteht darin, dass der zum funktionellen Hilfsstoff zeitlich verzögert freigesetzte Wirkstoff zu einer höheren Rate resorbiert wird.

Im Sinne dieser Erfindung wird der Begriff "pharmazeutische Zubereitung" synonym zu Grundmischung, Präkonzentrat, Formulierung oder Wirkstoffträgersystem verwendet und enthält neben einem oder mehreren Wirkstoffen pharmazeutische und/ oder funktionelle Hilfsstoffe, welche die Arzneistoffwirkung steuern bzw. unterstützen können, sowie möglicherweise weitere Grundstoffe, die für eine Herstellung von Arzneiformen, d.h. Darreichungsformen von Zubereitungen von Arzneistoffen, benötigt werden.

Unter Multilayerpellets werden erfindungsgemäß feste pharmazeutische Zubereitungen verstanden, die einen Pelletkern mit mindestens zwei aufeinanderfolgenden Schichten enthalten, wobei mindestens eine Schicht einen oder mehrere Wirkstoffe enthält.

Im Sinne dieser Erfindung beinhaltet der Begriff "Wirkstoff" Stoffe, die eine pharmakologische Hauptwirkung aufweisen und die ein Substrat von intestinalen metabolisierenden Enzymen sind.

Bevorzugte Wirkstoffe sind natürliche oder synthetische Estrogene und Estrogenderivate wie Estradiol, Estriol, Estrone sowie Derivate davon. Bevorzugt sind dabei Ethinylestradiol und dessen Derivate.

"Substrat mindestens eines intestinalen Enzyms" bedeutet erfindungsgemäß, dass Stoffe, insbesondere Wirkstoffe im Intestinum durch das jeweilige Enzym metabolisiert werden.

Unter "funktionellem Hilfsstoff' im Sinne der Erfindung ist ein pharmazeutischer Hilfsstoff zu verstehen, der weitgehend chemisch inert, von den Arzneimittelbehörden als solcher für die Verwendung in Arzneimitteln zugelassen ist und zugleich inhibitorische Wirkung auf mindestens eines der intestinalen metabolisierenden Enzyme besitzt. Unter weitgehend chemisch inert sollen auch solche Hilfsstoffe verstanden werden, die als Lösungsmittel, Lösungsbeschleuniger, Löslichkeitsvermittler, Emulgator, Solubilisator, Netzmittel, Antischaummittel oder Stabilisator in der pharmazeutischen Formulierung wirken. Weiterhin ist dieser funktionelle Hilfsstoff ein Inhibitor von mindestens einem der intestinalen metabolisierenden Enzyme. Insbesondere werden im Sinne der vorliegenden Erfindung unter funktionellen Hilfsstoffen Tenside, bevorzugt nichtionische Tenside, besonders bevorzugt polyethoxylierte Tenside, ganz besonders bevorzugt Polysorbate verstanden. Beispielsweise seien genannt: Polysorbat 20, 40, 60, 65, 80. Besonders bevorzugt ist Polysorbat 80 (Tween 80).

Im Sinne dieser Erfindung steht der Begriff "intestinales Enzym" für Enzyme, die unter anderem im intestinalen Gewebe vorkommen und dort die Absorption von pharmazeutischen Wirkstoffen durch metabolischen Abbau zumindest teilweise verhindern und damit die Bioverfügbarkeit nach peroraler Gabe vermindern können. Beispiele für intestinale Enzyme sind Enzyme des Phase I Metabolismus, beispielsweise aus der Oberfamilie der Cytochrom P450 Monooxygenasen, insbesondere CYP 3A4, CYP 2C9 und CYP 2C19, sowie Enzyme der Phase II wie Sulfotransferasen und Glucuronyltransferasen, beispielsweise SULT1 E1 und UGT1A1.

Im Sinne dieser Erfindung bedeutet "Inibition der intestinalen metabolisierenden Enzyme", dass in Anwesenheit der erfindungsgemäßen Zubereitung oder der funktionellen Hilfsstoffe, die für die erfindungsgemäße Zubereitung verwendet werden, Substrate von intestinalen Enzymen durch diese nur in geringerem Maße metabolisiert werden. Die Inhibition von intestinalen metabolisierenden Enzymsystemen kann mit dem Fachmann bekannten Testsystemen wie der Bestimmung der Inhibition des metabolischen Abbaus einer Wirksubstanz in intestinalen Geweben oder Präparationen daraus (Zellen, Mikrosomen, Cytochrom P450 Enzyme, etc.) bestimmt werden.

Unter Pelletkern im Sinne der Erfindung wird der gesamte innere Teil einer Mehrschicht(Multilayer)-Pelletformulierung unterhalb aller Überzugsschichten erfasst. Im Sinne dieser Erfindung ist Pelletkern definiert als neutraler Starterkern (Nonpareille), welcher als Trägermaterial in Form von kugelförmigen Granulatpartikeln vorliegt.

Für die Herstellung des neutralen Pelletkerns können verschiedene Materialien verwendet werden. Dazu zählen zum Beispiel Stärken, Cellulosen und Laktose (Milchzucker). Neben Kartoffel- oder Weizenstärke wird bevorzugt Maisstärke verwendet. Auch modifizierte Stärken und Stärkehydrolysate können zum Einsatz kommen. Im Sinne der Erfindung wird bevorzugt mikrokristalline Cellulose verwendet.

Der Durchmesser der Cellulose-Pelletkerne liegt erfindungsgemäß zwischen 0,1 und 1,5 mm, bevorzugt zwischen 0,2 und 0,4 mm. Die Dicke der darauf folgenden Schichten beträgt je Schicht zwischen 1 und 5 µm. Die Schichtdicke wird mit dem Fachmann bekannten Methoden, beispielsweise Rasterelektronenmikroskopie bestimmt.
Der Durchmesser der Pellets ist größer als 0,1 mm und kleiner oder gleich 2 mm. Der Durchmesser wird mit dem Fachmann bekannten Methoden, beispielsweise Lichtmikroskopie bestimmt.
Weiterhin bevorzugt ist eine Partikelgrößenverteilung der Pellets von 0,2 bis 0,4 mm. Die Partikelgrößenverteilung ist so, dass mindestens 85 % der Partikel einen Durchmesser von 0,2 bis 0,4 mm haben.

Die Oberfläche der Pelletkerne ist bevorzugt glatt, damit die aufzutragende Wirkstoffschicht nicht in den Pelletkern eindringt, sondern einen zusammenhängenden Film bildet.

Die über der Wirkstoffschicht gelegene Polymerschicht enthält erfindungsgemäß Polymere, die durch Diffusion des Wirkstoffs durch die Polymerschicht eine zum funktionellen Hilfsstoff zeitlich verzögerte Wirkstofffreisetzung steuern. Bevorzugt sind hierfür Polymere, die nach oraler Gabe im Gastrointestinaltrakt unlöslich aber quellbar sind und damit eine für den Wirkstoff permeable Membran bilden. Hierbei kommen insbesondere Polyvinylderivate (Kollicoat SR 30 D), Cellulosederivate (Ethylcellulose) sowie Polymethacrylate (Eudragit RS, Eudragit N 30 D, Eudragit L, Eudragit S) in Betracht. Besonders bevorzugt ist dabei das Polyvinylacetat Kollicoat SR 30 D. Bei Kollicoat SR 30 D handelt es sich um eine handelsübliche Suspension bestehend aus 27,0 % Polyvinylacetat, 2,7 % Povidon K30, 0,3 % Natriumlaurylsulfat und 70,0 % Wasser.

Die Freisetzung des Wirkstoffs durch die Polymerschicht kann durch die Dicke der Polymerschicht gesteuert werden. Eine Steuerung der Freisetzung durch verschiedene Anteile wasserlöslicher Porenbildner im wasserunlöslichen Polymer ist ebenfalls möglich. Als wasserlöslicher Porenbildner kommen zum Beispiel Polyethylenglykol (PEG), Polyvinylpyrrolidon (PVP, z.B. Kollidon®-Typen der Firma BASF), Polyvinylalkohol-Polyethylenglykol-Copolymere (PVA-PEG-Copolymere, z.B. Kollicoat® IR-Typen der Firma BASF) oder deren Derivate Betracht.

Das oben beschriebene Multilayer-Pellet zeichnet sich durch eine pH-unabhängige, zum funktionellen Hilfsstoff zeitlich verzögerte Wirkstofffreisetzung aus. Eine pHabhängige Auflösung der Polymermembran ist ebenfalls möglich. In Betracht kommen dafür magensaftresistente, aber dünndarmlösliche Polymere, die vom sauren Magenmilieu nicht angegriffen werden, jedoch im schwach sauren, neutralen oder alkalischen Milieu des Dünndarms aufgelöst werden. Derartige Polymere weisen eine freie Säurefunktion in der Seitenkette auf, die in der Salzform gut wasserlöslich ist. Dazu zählen beispielsweise Cellulosederivate (Carboxymethylethylcellulose, Celluloseacetatphthalat, Hydroxypropylmethylcellulosephthalat), Polymethacrylate (Eudragit L 100, Eudragit S 100, Eudragit L 100-55), Polyvinylderivate (Polyvinylacetatphthalat) sowie Polyhydroxycarbonsäuren (Schellack). Durch die Dicke der Polymerschicht und durch die Wahl des Polymers sind auch hier Möglichkeiten für zeitlich gesteuerte Freisetzungsprozesse gegeben.

Eine Zeitverzögerung der Wirkstofffreigabe kann auch durch eine langsame Wirkstoffauflösung durch Anwendung von Makrokristallen, anderen Kristallmodifikationen oder schwerlöslichen Salzen erreicht werden.

Ein weiteres Prinzip der Freisetzungssteuerung kann auch mit aufplatzenden Polymermembranen erreicht werden. Bei oral verabreichten Arzneiformen mit permeablen Überzügen strömt Flüssigkeit durch den Polymerfilm hindurch. Ein dabei entstehender hoher Innendruck bewirkt ein Aufreißen der Hülle und somit die Freisetzung des Wirkstoffs. Steuerungsgröße für die Zeit bis zum Aufplatzen der Polymermembran stellt neben der Permeabilität für Wasser vor allem die Schichtdicke der Polymermembran dar.

Zur Freisetzungssteuerung des Wirkstoffes aus pharmazeutischen Formulierungen kann der Wirkstoff auch in Matrices eingebettet werden. So kann anstelle von neutralen Starterpellets, welche mit einer Wirkstoffschicht überzogen werden, der Wirkstoff auch direkt in den Pelletkern eingebettet werden. Als Matrix kommen dafür beispielsweise Polymilchsäure, Polyamide, Polyanhydride, Polyglutaminsäuren und Polyaminsäuren in Betracht. Die Löslichkeit des Wirkstoffs in der Matrix ist Voraussetzung für die Freisetzung. Ferner können unterschiedliche Anteile an Porenbildner in der Matrix das Freisetzungsverhalten des Wirkstoffs steuern.

Alternativ dazu können auch auf Fetten basierte bioabbaubare Systeme Anwendung finden. Es erfolgt eine Erosion, d.h. eine auf einem allmählichen Abbau, gegebenenfalls auch einer Auflösung der Arzneiform beruhende Verringerung der Diffusionsbarriere. In Betracht kommen synthetische Triglyceride (Fettpellets), die im Gastrointestinaltrakt durch Lipasen abgebaut werden. Über die Wahl des Triglycerids kann die Freisetzungsgeschwindigkeit des Wirkstoffs gesteuert werden. Die enzymatische Esterhydrolysegeschwindigkeit ist bei kurzkettigen Glyceriden schneller, bei langkettigen Glyceriden langsamer.

Alternativ zu einem Multilayer-Pellet ist auch die Mischung verschiedener Pelletpopulationen denkbar. Das heißt, dass eine Pelletpopulation den funktionellen Hilfsstoff enthält, welcher sofort freigesetzt wird. Eine zweite Pelletpopulation enthält den Wirkstoff, der gezielt verzögert zum funktionellen Hilfsstoff abgegeben wird.

Die Pellets können in Kapseln abgefüllt oder als Tablette verpresst werden, die jeweils nach oraler Applikation schnell wieder in ihre Untereinheiten - den Einzelpellets - zerfällt.

Weiterhin können die wirkstoffhaltigen Pellets in Kapseln abgefüllt werden, wobei die Kapseln mit einer Schicht umhüllt werden, die einen funktionellen Hilfsstoff enthält.

Die Kapseln können auch zugleich wirkstoffhaltige Pellets sowie eine den funktionellen Hilfsstoff enthaltene Lösung umfassen.

Eine weitere Möglichkeit besteht in der Herstellung von Manteltabletten alternativ zu Pelletformulierungen. Dabei wird ein wirkstoffhaltiger Tablettenkern (wirkstoffhaltige Matrix) von einem den funktionellen Hilfsstoff enthaltenden Mantel/ Polymerfilm umschlossen.

Auch osmotische Systeme können formuliert werden. Diese nach dem Prinzip einer osmotischen Pumpe arbeitende Arzneiform besteht aus einem Wirkstoff-Hilfsstoff-Reservoir das von einer semipermeablen Kunstoffmembran umgeben ist. In letzterer befindet sich ein mikrofeines Loch. Dringt Wasser in das Reservoir ein, wird der Wirkstoff aufgrund des osmotischen Drucks im Innern über die Abgabeöffnung konstant freigesetzt. Denkbar sind Zweikammer-Systeme. Eine Kammer setzt schnell den funktionellen Hilfsstoff, die andere Kammer verzögert dazu den Wirkstoff frei.

Im Sinne der Erfindung wird unter zeitversetzter Freisetzung oder zeitlich verzögerter Freisetzung verstanden, dass
- der funktionelle Hilfsstoff zu mindestens 70 % in 20 min, bevorzugt mindestens 75 % in 15 min, besonders bevorzugt mindestens 80% in 10 min sowie
- der Wirkstoff nach 1 Stunde zwischen 30 und 70 %, bevorzugt zwischen 40 und 60 %, nach 2 Stunden zu mehr als 50 %, bevorzugt mehr als 70 % aus der pharmazeutischen Formulierung freigesetzt wird.

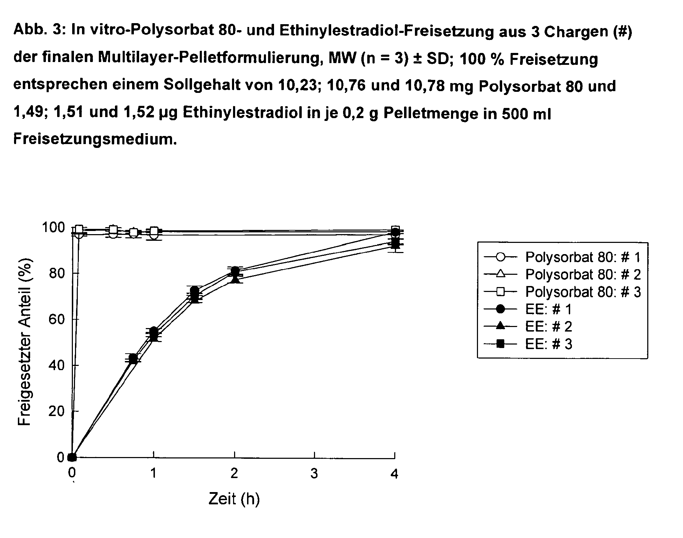

Die Freisetzung des funktionellen Hilfsstoffes sowie des Wirkstoffes wird im Freisetzungstest in 500 ml Phosphatpuffer bei einem pH-Wert von 6.8 im Dissolutiontester mit Peak-Vessel bei 75 rpm bestimmt.
Dies bedeutet in anderen Worten, dass zwischen dem gemessenen oder intrapolierten Zeitpunkt innerhalb eines Freisetzungstests in 500 ml Phosphatpuffer bei einem pH-Wert von 6.8 im Dissolutiontester mit Peak-Vessel bei 75 rpm, zu dem 70 % des funktionellen Hilfsstoffes freigesetzt sind, und dem gemessenen oder intrapolierten Zeitpunkt innerhalb desselben Freisetzungstests, zu dem 70 % des Wirkstoffes freigesetzt sind, eine Verzögerungszeit von 0,25 bis 3 Stunden, bevorzugt von 0,5 bis 2 Stunden, besonders bevorzugt von 1 bis 1,5 Stunden liegt.
Ein Beispiel ist in Abbildung 3 dargestellt.

Die Fähigkeit von Hilfsstoffen, intestinale metabolisierende Enzyme wie humane Cytochrom P450 Isoenzyme zu inhibieren, wird über einen CYP-Test festgestellt (Assayprinzip dargestellt in Krippendorff et al., J. Biomol. Screen. 2007, 12(1):92-9). Die Inhibierung der Enzyme wird hierbei über die Konzentration (IC₅₀, µg/ mL) der Hilfsstoffe charakterisiert, bei der 50 % der jeweiligen Isoenzyme inhibiert werden. Es hat sich gezeigt, dass die CYP-Isoenzyme, insbesondere CYP8A4, durch die erfindungsgemäßen Hilfsstoffe, insbesondere Polysorbat 80, selektiv inhibiert werden. Die erfindungsgemäßen Hilfsstoffe weisen an mindestens einem CYP-Isoenzym mindestens eine IC₅₀ < 1000 µg/ mL auf, mit Vorteil eine IC₅₀ ≤ 100 µg/ mL, d.h. sie besitzen mittlere Aktivität, und mit besonderem Vorteil eine IC₅₀ ≤ 10 µg/ mL, d.h. sie besitzen ein starkes inhibitorisches Potential auf das untersuchte Isoenzym (z.B. CYP 3A4, vgl. Tab 1).

**Tabelle 1:**

| Inhibierungsaktivitäten von Hilfsstoffen bzgl. CYP-Isoenzymen. | | | | |
|---|---|---|---|---|
| | | **IC50, µg/ml** | | |
| **Name** | **Stoff** | **CYP-2C9** | **CYP-2C19** | **CYP-3A4** |
| Cremophor^{®}EL | Polyethylenglycol-35-Rizinusöl | 2,1 | 10 | 16 |
| Cremophor^{®}RH 40 | PEG-40-hydriertes Rizinusöl | 35 | 11 | 23 |
| Estax^{®}54 | PEG-400-monoricinoleat | 1,6 | 0,7 | 3,8 |
| Miglyol^{®}812 | C8-C12 Triglyceride (MCT) | 350 | 100 | 245 |
| raff. Rizinusöl | raffiniertes Rizinusöl (RIZ) | 1150 | >3000 | 930 |
| Transcutol^{®}P | Ethoxydiglycol | 1500 | 1400 | 755 |
| Ethyloleat | Ethyloleat (EO) | 195 | 325 | 117 |
| Imwitor^{®}308 | Glycerolmonocaprylat | 5,0 | 6,9 | 29 |
| Tween^{®}80 | Polysorbat 80 | 2,9 | 8,4 | 7,3 |

Diese Ergebnisse belegen, dass erfindungsgemäße pharmazeutische Zubereitungen intestinale Enzyme, insbesondere Cytochrom-Isoenzyme wie CYP3A4 inhibieren und somit zu einer Erhöhung der Bioverfügbarkeit von Arzneistoffen führen können.

**Herstellung der erfindungsgemäßen pharmazeutischen Pelletformulierung** Pellets können in Dragierkesseln, Wirbelschichtapparaturen oder speziellen Pelletiereinrichtungen nach dem Fachmann bekannten Methoden hergestellt werden.

### Allgemeine Vorschrift zur Herstellung der erfindungsgemäßen Multilayerpellets:

Für die erfindungsgemäßen Multilayerpellets wurden Pelletkerne der Firma Syntapharm (Mülheim an der Ruhr, Deutschland), verwendet.
Der neutrale Pelletkern mit einem mittleren Partikeldurchmesser von 200 - 355 µm besteht aus mikrokristalliner Cellulose als Trägermaterial für einen Mehrschichtaufbau. Auf diese Pelletkerne wurden schrittweise drei Schichten aufgetragen (von innen nach außen): 1. eine Wirkstoffschicht (Ethinylestradiol), 2. ein die Freisetzung kontrollierendes Polymer für die verzögerte Freisetzung des darunter liegenden Wirkstoffs sowie 3. eine äußere, eine den Hilfsstoff enthaltene Schicht, die diesen dann schnell freisetzt. Schnelle Freisetzung bedeutet hierbei, dass der funktionelle Hilfsstoff (bestimmt in 500 ml Phosphatpuffer bei pH 6.8 im Dissolutiontester mit Peak-Vessel bei 75 rpm) mindestens zu 70 % in 20 min, bevorzugt mindestens 75 % in 15 min besonders bevorzugt mindestens 80 % in 10 min, ganz besonders bevorzugt im wesentlichen vollständig innerhalb von 5 Minuten freigesetzt wird.

sowie 3. eine äußere, eine den Hilfsstoff enthaltene Schicht, die diesen dann innerhalb von 5 Minuten freisetzt.

### Beispiel

### A) Schicht 1: Wirkstoffhaltiges Coating (Ethinylestradiol)

Der Wirkstoff Ethinylestradiol wurde mittels eines Hydroxypropylmethylcellulosefilms auf die Starterpellets aufgetragen. Dazu wurde HPMC (Methocel E5 Premium LV) in der auf 60 °C erwärmten Wassermenge eingearbeitet und unter moderatem Rühren mittels Magnetrührer klar gelöst. Nach Abkühlen der Polymerlösung wurde ein Volumenaliquot einer zuvor hergestellten Ethinylestradiol-Stammlösung (Lösungsmittelgemisch Wasser/ Ethanol im Verhältnis 1 : 1) zupipettiert und homogenisiert. Abschließend wurde das durch Erwärmen verdunstete Wasser ergänzt. Die Zusammensetzung der Wirkstoffhaltigen Schicht ist in der Tab. 2 aufgeführt.

**Tab. 2: Zusammensetzung des Ethinylestradiol-haltigen Coatings.**

| **Substanz** | **Masse (g)** | **Anteil (%)** |
|---|---|---|
| Ethinylestradiol, absolut [als Stammlösung: 5 mg EE in 10 ml H₂O:Ethanol (1:1)] | 0,005 | 0,001 |
| Methocel E5 Premium LV | 6,0 | 2,0 |
| gereinigtes Wasser | 294,0 | 98,0 |
| gesamt | 300,0 | 100,0 |

### B) Schicht 2: Freisetzungskontrollierendes Polymercoating (Polyvinylacetat)

Zur Herstellung von Polyvinylacetat-Coatings wurde Polyvinylacetat als handelsübliche Polymerdispersion Kollicoat ® SR 30 D sowie der Weichmacher Propylenglykol nacheinander in das vorgelegte Wasser unter Rühren hinzugefügt. In einem separaten Gefäß wurde mikronisiertes Talkum in Wasser eingearbeitet und mittels Ultra-Turrax^{®} homogenisiert. Die Talkumsuspension wurde in einem getrennten Schritt hergestellt, um mechanische Scherkräfte durch den Ultra-Turrax auf das Polymer zu vermeiden. Die Talkumsuspension wurde in die Polymersuspension mittels Propellerrührer eingearbeitet und homogenisiert. Die Zusammensetzung der Polymer-Schicht ist in der Tab. 3 aufgeführt.

**Tab. 3: Zusammensetzung der freisetzungskontrollierenden Coatings auf Basis von Polyvinylacetat (Kollicoat® SR 30 D) in verschiedenen Schichtdicken (bezogen auf Polymermasse pro Pelletoberfläche).**

| **Substanz** | **Masse (g)** |
|---|---|
| *Coatingschichtdicke:* | *0, 5 mg*/*cm*² |

| *Polymersuspension:* | |
|---|---|
| Kollicoat^{®} SR 30 D*⁾ | 140,9 |
| 1,2-Propylenglykol**⁾ | 4,3 |
| gereinigtes Wasser | 98,8 |

| *Talkumsuspension:* | |
|---|---|
| Talkum, mikronisiert | 16,9 |
| gereinigtes Wasser | 25,5 |
| gesamt | 286,4 |

| | |
|---|---|
| * 30 %ige wässrige Dispersion von Polyvinylacetat, stabilisiert mit 2,7 % Povidon K30 und 0,3 % Natriumlaurylsulfat, ** 10 % Weichmacher bezogen auf den Polymerfeststoffanteil. | |

### C) Schicht 3: Hilfsstoffhaltiges Coating (Polysorbat 80 = Tween 80)

Wasser wurde auf 60 °C erwärmt und der funktionelle Hilfsstoff Polysorbat 80 unter Rühren darin gelöst. Anschließend wurde die HPMC (Methocel E5 Premium LV) zur Lösung gegeben und ebenfalls unter Rühren gelöst. Nach Abkühlen der Lösung wurde das verdunstete Wasser ergänzt. Die Zusammensetzung der Hilfsstoff-haltigen Schicht ist in der Tab. 4 aufgeführt.

**Tab. 4: Zusammensetzung des Polysorbat 80-haltigen Coatings.**

| **Substanz** | **Masse (g)** | **Anteil (%)** |
|---|---|---|
| Polysorbat 80 | 25,0 | 10,0 |
| Methocel E5 Premium LV | 18,75 | 7,5 |
| gereinigtes Wasser | 206,25 | 82,5 |
| gesamt | 250,0 | 100,0 |

### D) Coating der Pellets

Das Coating der Pellets wurde im Wirbelschichtgerät mit Wurster-Zylinder unter Verwendung des Bottom-Spray-Verfahrens durchgeführt. Durch Regulierung des Zuluftstroms auf eine Luftmenge von 30 - 35 m³/h entsteht ein gleichmäßiges Strömungsbild der Pellets mit einem durchschnittlichen Teilchendurchmesser von 200 - 355 µm. Die Regulierung des Zuluftstroms kann beispielsweise durch den Einsatz einer Lochbodenplatte mit eng verteilten, kleinen Perforationen realisiert werden. Es wurde mit einer Sprühdüse von 0,8 mm Durchmesser bei einem Sprühdruck von 2,5 bar gearbeitet. 500 g Pellets wurden im Wirbelschichtgerät mit der für das jeweilige Coating entsprechenden Zulufttemperatur 2 - 3 min vorgewärmt und anschließend mit der entsprechenden Polymerlösung bzw. Polymersuspension überzogen. Die Sprührate der Coatingrezeptur wurde durch die Förderung mittels einer Schlauchpumpe so angepasst, dass sich die gewünschte Produkttemperatur einstellte (in Anlehnung an technische Informationen der Polymerhersteller) und Agglomerationen der Pellets verhindert wurden. Weitere Prozessparameter können Tab. 4 entnommen werden. Nach dem Erreichen der theoretisch benötigten Auftragsmenge wurde der Sprühvorgang beendet und die Pellets abschließend in der Wirbelschicht 2 min ohne weitere Wärmezufuhr nachgetrocknet. Im Trockenschrank wurden die Pellets 24 h bei 40 °C thermisch nachbehandelt.

**Tab. 5: Prozessparameter für die verschiedenen Coatings in der Wirbelschicht.**

| **Rezeptur** | **Schicht 1** **(Wirkstoff)** | **Schicht 2** **(Polymer)** | **Schicht 3** **(Hilfsstoff)** |
|---|---|---|---|
| Zulufttemperatur | 50 °C | 45 °C | 50 °C |
| Produkttemperatur | 25 - 27 °C | 25 - 28 °C | 30 - 33 °C |
| Sprührate | 4,7 g/min | 4,8 g/min | 2,9 g/min |
| Sprühzeit | 64 min | 60 min | 85 min |

### Mehrschichtiger Aufbau der Pellets

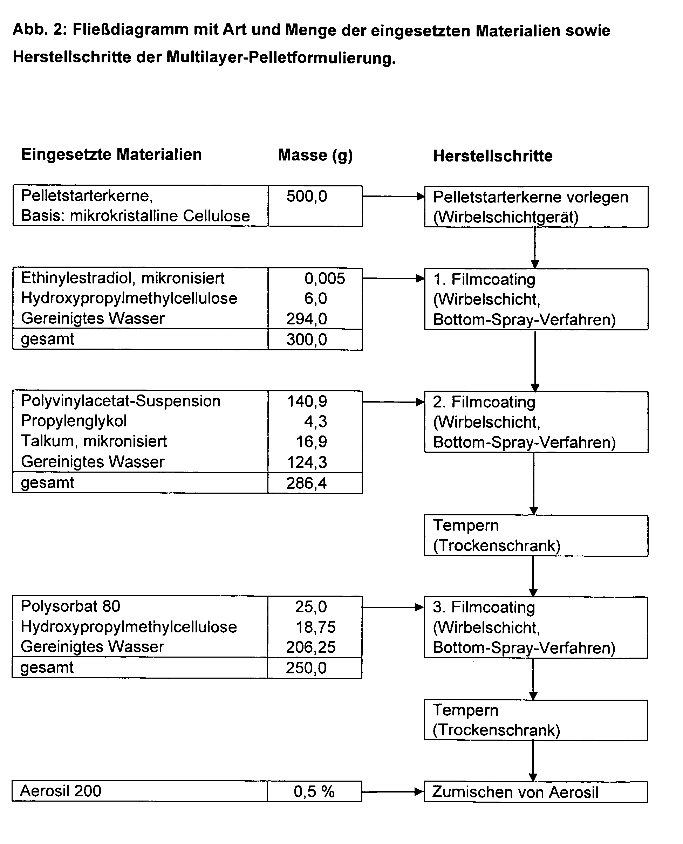

Um die Pellets mit einer weiteren Schicht zu überziehen, wurde wie unter Beispiel 1 D) beschrieben verfahren. Erneut wurden die Pellets in der Wirbelschicht vorgewärmt und die berechnete Coatingsuspension bzw. -lösung aufgesprüht. Auch hier folgte nach Trocknung der Pellets in der Wirbelschicht wieder eine endgültige Verfilmung durch Tempern im Trockenschrank. Zur Verbesserung der Fließfähigkeit kann der Pelletmenge abschließend 0,5 % Aerosil zugesetzt werden. Die quantitative Zusammensetzung aller drei Schichten sowie der schrittweise Ablauf des Prozesses sind in Abbildung 2 zusammengetragen worden.

### Charakterisierung der Pellets:

### 1. Gehaltsbestimmung von Ethinylestradiol

Um den Gehalt sowie dessen Gleichförmigkeit von Ethinylestradiol in den Pelletformulierungen zu bestimmen, wurden jeweils 10 Pelletportionen pro Formulierung zu je 1,0 g in Messkolben mit je 10,0 ml Lösungsmittelgemisch (Acetonitril : Methanol : Wasser im Verhältnis 6 : 1 : 3 (m/m)) versetzt. Nach 10-minütiger Behandlung der Proben im Ultraschallbad wurden diese in Zentrifugengläser überführt und bei einer Zentrifugalbeschleunigung von ≥ 2000 g für 10 min zentrifugiert. Die klaren überstehenden Lösungen wurden in HPLC-Vials überführt und mittels HPLC und Fluoreszenzdetektion quantifiziert.

### 2. Gehaltsbestimmung von Polysorbat 80

Um den Gehalt des funktionellen Hilfsstoff Polysorbat 80 in den Pellets zu bestimmen, wurde in gleicher Weise wie bei der Gehaltsbestimmung von Ethinylestradiol verfahren. Einziger Unterschied in der Probenaufarbeitung bestand darin, dass die Pellets mit Wasser anstelle von organischen Lösungsmitteln versetzt wurden. Für die Anaiytik wurden die Probenlösungen noch einmal 1 : 50 mit Wasser verdünnt (linearer Bereich der Polysorbat 80-Analytik). Die klaren überstehenden Lösungen wurden in HPLC-Vials überführt und mittels HPLC und Charged Aerosol-Detektion quantifiziert.

### 3. Freisetzung von Ethinylestradiol

Zur Bestimmung der Freisetzung des Wirkstoffs Ethinylestradiol aus den Pelletformulierungen wurden In vitro-Freisetzungsversuche nach den folgenden Prüfbedingungen durchgeführt.

**Tab. 6: Prüfbedingungen für die Freisetzungsmethode.**

| | |
|---|---|
| Gerät: | Freisetzungsapparatur App. 2 : 6 Peak^{™}-Vessel mit Deckel und 6 Paddle-Rührvorrichtungen |
| Medium: | Phosphatpuffer pH 6,8 (nach USP) + Zusatz von 0,1 mg/ml Polysorbat 80 |
| Füllvolumen: | 500 ml |
| Temperatur: | (37 ± 0,5) °C |
| Rührgeschwindigkeit: | 75 U/min |
| Probenmenge: | 200 mg Pellets |

Beim Peak^{™}-Vessel handelt es sich um ein Freisetzungsgefäß, bei dem sich im halbkugelförmigen Boden eine Einbuchtung - "Cone" befindet. Das Probenmaterial wird in der Region des Vessels platziert, in der das Medium in moderater Bewegung mit gleichmäßiger Hydrodynamik ist.

Es erfolgte eine Dreifachbestimmung pro Formulierung. Das Freisetzungsmedium wurde auf 37 °C temperiert, bevor die zu untersuchende Pelletmenge gleichmäßig in das Probengefäß eingestreut wurde. Die Probeentnahmezeitpunkte wurden angepasst an die Formulierung gewählt. Für schnell freisetzende Bestandteile (funktioneller Hilfsstoff) wurden nach jeweils 5, 10, 20, 30, 45 und 60 min Proben entnommen, bei verzögerter Freigabe (Wirkstoff) wurden Proben nach jeweils 30, 45, 60, 90 min sowie nach 2 und 4 h gezogen. Je 10 ml der Prüflösungen wurden dem Probengefäß mit einer Injektionsspritze entnommen, über einen 0,45 µm Membranfilter, regenerierte Cellulose, filtriert und in HPLC-Vials zur quantitativen Bestimmung von Polysorbat 80 sowie Ethinylestradiol überführt. Das entnommene Volumen wurde nach den Probenzügen nicht wieder aufgefüllt, sondern rechnerisch bei der Auswertung berücksichtigt.

### 4. Freisetzung von Polysorbat 80

Zur Bestimmung der Freisetzung des funktionellen Hilfsstoffs Polysorbat 80 aus den Pelletformulierungen wurde in gleicher Weise wie bei der Bestimmung der Freisetzung von Ethinylestradiol verfahren. Einziger Unterschied in der Durchführung bestand darin, dass dem Freisetzungsmedium kein zusätzliches Polysorbat 80 zugesetzt wurde.

### Ergebnisse:

Nach Gehaltsbestimmung von Polysorbat 80 und Ethinylestradiol in drei aufeinanderfolgend hergestellten Chargen, Tab. 7, wurde das Freisetzungsverhalten bestimmt.

**Tab. 7: Gehaltsbestimmung von Polysorbat 80 und Ethinylestradiol auf 200 mg der Multilayer-Pelletformulierung von drei Chargen.**

| **Pelletformulierung** | **Gehalt Polysorbat 80 (mg)** | **Gehalt EE (µg)** |
|---|---|---|
| Charge 1 | 10,23 | 1,49 |
| Charge 2 | 10,76 | 1,51 |
| Charge 3 | 10,78 | 1,52 |

Die Freisetzungskurven von Polysorbat 80 und Ethinylestradiol der drei untersuchten Pelletchargen legen in Abb. 3 reproduzierbare Freisetzungsergebnisse der Multilayer-Pelletformulierung dar.

## Patentansprüche

1. Feste pharmazeutische Zubereitung zur oralen Applikation in Form von Multilayerpellets, die, von innen nach außen gesehen:
a) einen Pelletkern,
b) eine Schicht, enthaltend mindestens einen Wirkstoff,
c) eine darüber gelegene Schicht, enthaltend mindestens ein Polymer und
d) eine äußere Schicht, enthaltend mindestens einen funktionellen Hilfsstoff
enthält.

2. Multilayerpellets gemäß Anspruch 1, **dadurch gekennzeichnet, dass** der Wirkstoff ein Estrogen oder Estrogenderivat ist.

3. Multilayerpellets gemäß Anspruch 2, **dadurch gekennzeichnet, dass** der Wirkstoff Ethinylestradiol ist.

4. Multilayerpellets gemäß Ansprüche 1-3, **dadurch gekennzeichnet, dass** der funktionelle Hilfsstoff ein Tensid, bevorzugt ein nichtionisches Tensid ist.

5. Multilayerpellets gemäß Anspruch 4 **dadurch gekennzeichnet, dass** der funktionelle Hilfsstoff ein polyethoxyliertes Tensid, bevorzugt ein Polysorbat ist.

6. Multilayerpellets gemäß Anspruch 4 und 5 **dadurch gekennzeichnet, dass** der funktionelle Hilfsstoff Polysorbat 80 (Tween 80) ist.

7. Multilayerpellets gemäß Ansprüche 1-6, **dadurch gekennzeichnet, dass** die Polymere der mittleren (2.) Schicht die Wirkstoffe zeitlich verzögert freisetzen.

8. Multilayerpellets gemäß Anspruch 7, **dadurch gekennzeichnet, dass** die Polymere wasserunlöslich, aber quellbar sind.

9. Multilayerpellets gemäß Anspruch 8, **dadurch gekennzeichnet, dass** die Polymere den Wirkstoff unabhängig vom pH-Wert freisetzen.

10. Multilayerpellets gemäß Anspruch 9, **dadurch gekennzeichnet, dass** die Polymerschicht Polyvinylacetat, bevorzugt das Polyvinylacetat Kollicoat SR 30D enthält.

11. Multilayerpellets gemäß Ansprüche 1-10, **dadurch gekennzeichnet, dass** der mittlere Pelletdurchmesser zwischen 0,2 und 0,4 mm liegt.

12. Multilayerpellets gemäß Anspruch 1-11 zur Verwendung in der weiblichen Fertilitätskontrolle.

13. Multilayerpellets gemäß Anspruch 1-11 zur Verwendung in der Hormonersatztherapie.

14. Multilayerpellets gemäß Anspruch 1-11 zur Herstellung eines Arzneimittels.

15. Kapsel oder Tablette enthaltend Multilayerpellets gemäß Anspruch 1-11.
